**Europäisches Patentamt**

**European Patent Office**

**Office européen des brevets**

(19)

(11) Veröffentlichungsnummer : **0 456 764 B1**

(12) # EUROPÄISCHE PATENTSCHRIFT

(45) Veröffentlichungstag der Patentschrift :
**01.09.93 Patentblatt 93/35**

(21) Anmeldenummer : **90903830.9**

(22) Anmeldetag : **31.01.90**

(86) Internationale Anmeldenummer :
**PCT/EP90/00170**

(87) Internationale Veröffentlichungsnummer :
**WO 90/08543 09.08.90 Gazette 90/19**

(51) Int. Cl.$^5$ : **A61K 31/20**, A61K 31/23,
A61K 9/00, A61K 9/107,
A61K 47/24

(54) **OMEGA-3-FETTSÄURENHALTIGE FETTEMULSION ZUR ENDOTRACHEALEN APPLIKATION, IHRE HERSTELLUNG UND ANWENDUNG.**

(30) Priorität : **02.02.89 DE 3903057**

(43) Veröffentlichungstag der Anmeldung :
**21.11.91 Patentblatt 91/47**

(45) Bekanntmachung des Hinweises auf die
Patenterteilung :
**01.09.93 Patentblatt 93/35**

(84) Benannte Vertragsstaaten :
**AT BE CH DE DK ES FR GB IT LI LU NL SE**

(56) Entgegenhaltungen :
**EP-A- 298 293**
**EP-A- 311 091**

(73) Patentinhaber : **B. Braun Melsungen AG**
**Postfach 120**
**D-34209 Melsungen (DE)**

(72) Erfinder : **GEORGIEFF, Michael**
**An der Lehrten 5**
**D-7834 Herbolzheim (DE)**
Erfinder : **NEHNE, Jörg**
**Wesserring 7**
**D-3501 Guxhagen (DE)**
Erfinder : **BOLL, Michael**
**Liepziger Strasse 6**
**D-3508 Melsungen (DE)**

(74) Vertreter : **von Kreisler, Alek, Dipl.-Chem. et al**
**Patentanwälte Von Kreisler-Selting-Werner,**
**Postfach 10 22 41, Bahnhofsvorplatz 1**
**D-50462 Köln (DE)**

EP 0 456 764 B1

Jouve, 18, rue Saint-Denis, 75001 PARIS

EP 0 456 764 B1

## Beschreibung

Die vorliegende Erfindung betrifft die Verwendung einer Fettemulsion zur Herstellung eines Arzneimittels zur endotrachealen Behandlung von Lungenerkrankungen.

Das Lungenversagen ist die lebensbedrohliche Folge verschiedener pulmonaler Affektionen und extrapulmonaler Erkrankungen wie Pneumonie, Aspiration, inhalative Noxen, Schock, Sepsis, Trauma, Verbrennung, Intoxikationen und anderem.

Den auslösenden Uraschen ist gemeinsam, daß sie die klassischen Kaskaden-Systeme (Kallikrein-Kinin-System, Komplement-System, Gerinnungs-System) aktivieren. Im Zuge dieser Aktivierung werden Intermediärprodukte und Endprodukte gebildet, die allesamt den Arachidonsäurestoffwechsel aktivieren und zur Bildung von Prostaglandinen, Thromboxanen und Leukotrienen anregen. Diese zusammenfassend als Eikosanoide bezeichneten Mediatoren bilden aufgrund ihrer vasoaktiven Eigenschaften sowie wegen ihres

Einflusses auf Entzündung und Gefäßpermeabilität und in Kombination mit anderen Faktoren eine der Hauptursachen für das Lungenversagen bzw. andere entzündliche, traumatische und allergische Lungenerkrankungen. Außer den genannten Kaskaden-Systemen wirken auch alveoläre Hypoxie und unspezifische Reize verschiedenster Art (Embolie, Histamin, Toxine) als Stimulatoren des Eikosanoidsystems, indem sie Arachidonsäure aus pulmonalen Membranphospholipiden freisetzen. Dies unterstreicht die entscheidende Rolle der Arachidonsäure und ihrer Folgeprodukte als Endstreckenmediatoren des Lungenversagens (H. Neuhof, Medizinische Welt $\underline{35}$, (1984) 1457-1467).

Unmittelbar nach ihrer Freisetzung aus Membranphospholipiden entstehen aus der Arachidonsäure Prostaglandine und Thromboxane; von diesen sind das vasokonstriktorische und proaggregatorische $TXA_2$ und das zu einer pulmonalen Gefäßverengung führende $PGF_{2a}$ als wesentliche Faktoren bei der für die Pathogenese des Lungenversagens bedeutsamen Zunahme des pulmonalen Strömungswiderstands bekannt. Weitere Folgeprodukte des stimulierten Arachidonsäurestoffwechsels sind die Leukotriene, die z.T. die Gefäßpermeabilität erhöhen und auf diese Weise die Bildung von Lungenödemen fördern.

Die zentrale Rolle, die die Arachidonsäure in der Pathogenese von Lungenerkrankungen einnimmt, prädestiniert sie zu einem der Hauptangriffspunkte möglicher therapeutischer Interventionen. Hemmstoffe der Phospholipasen, also der Arachidonsäurefreisetzung, Hemmstoffe der Lipoxygenasen und/oder der Zyklooxygenasen, also der Leukotrien- und/oder Prostaglandinsynthese, sind in diesem Zusammenhang zu nennen und wurden mit unterschiedlichem Erfolg bereits zur Prophylaxe bzw. Therapie des Lungenversagens eingesetzt.

Die EP-A-0 298 293 betrifft eine Fettemulsion, die ein speziell hergestelltes und zusammengesetztes hochgereinigtes Fischöl enthält, das für parenterale und diätetische Zwecke verwendet wird. Gemäß dem einzigen Ausführungsbeispiel dieser Druckschrift wird unter parenteraler Verabreichung ausschließlich eine intravenöse Applikation verstanden.

In jüngster Zeit wurde außerdem vorgeschlagen, die Wirkungen der Arachidonsäure, die zu den $\omega$-6-Fettsäuren zählt, durch $\omega$-3-Fettsäuren, zu deren Hauptvertretern Eikosapentaensäure und Dokosahexaensäure gehören, und die natürlicherweise hauptsächlich in Fischölen vorkommen, zu antagonisieren.

Das US-Patent 4 526 902 beschreibt Mischungen aus 25 bis 75 Gew.-% Eikosapentaensäure und/oder Dokosahexaensäure und einer $\omega$-6-Fettsäure, die enteral als Bestandteil von Pharmazeutika oder fetthaltigen Nahrungsmitteln wie Butter oder ähnlichem verwendet werden.

In der EP 0 120 169 B1 sind synthetische Triglyzeride beschrieben, die am mittleren C des Glyzerinmoleküls eine mehrfach ungesättigte Fettsäure, vorzugsweise Eikosapentaensäure oder Dokosahexaensäure, besitzen können. Die so erzeugten Glyzeride können als Nahrungsmittel, Nahrungsergänzungsmittel oder als Arzneimittel für die therapeutische Ernährung verwendet werden.

Die JP-OS Sho-58-230918 beschreibt eine Eikosapentaensäure enthaltende Emulsion zur oralen und nicht-oralen Verwendung. Diese enthält 1 bis 40 w/v-% Eikosapentaensäure und/oder Dokosahexaensäure bzw. vorzugsweise deren Methyl- oder Ethylester, 1 bis 30 w/v-% eines Pflanzenöls, vorzugsweise Sojaöl, 0,01 bis 30 w/v-% $\alpha$-Tocopherol, und als Emulgatoren 0,1 bis 5 w/v-% eines Phospholipids, vorzugsweise aus Eigelb und/oder Soja, sowie 0,1 bis 10 w/v-% eines nichtionischen synthetischen Emulgators.

Die DE-OS 34 09 793 offenbart eine flüssige Emulsion zur Transfusion mit antithrombotischer und antiarteriosklerotischer Wirkung, die zur Nahrungsergänzung dienen kann. Sie besteht neben Wasser zu 5 bis 20 w/v-% aus Eikosapentaensäure, Dokosahexaensäure oder deren Ester und sie ist vorzugsweise ein gereinigtes Fischöl, wie Sardinenöl. Weiterhin enthält sie 1 bis 19 w/v-% eines Pflanzenöls, vorzugsweise Soja- und-/ oder Safloröl, sowie 1 bis 2 w/v-% eines Phospholipidemulgators, vorzugsweise aus Eigelb oder Soja. Dieser Fettemulsion kann als Antioxidans $\alpha$-Tocopherol zugefügt werden.

WO 87/02247 beschreibt Fettemulsionen, enthaltend Fischöle mit hohem Anteil an $\omega$-3-Fettsäureestern, die zur intravenösen Anwendung bei der Behandlung thrombotischer Erkrankungen vorgesehen sind.

In der EP-0 271 909 wird die Herstellung synthetischer Triglyzeride beschrieben, die als Fettsäuren solche

2

mit einer Kettenlänge von 6 bis 12 C-Atomen, 14 bis 18 C-Atomen (Vorzugsweise Linolsäure) sowie 20 bis 22 C-Atomen (vorzugsweise Eikosapentaensäure) in variablen Kombinationen und Relationen enthalten.

SE 8705122-A0 beschreibt ein Verfahren zur Herstellung einer intravenösen Fettemulsion, die $\omega$-3-Fettsäuren als Bestandteil des Emulgators enthält.

Die DE-OS 37 34 147 betrifft eine isotone Fettemulsion zur parenteralen Applikation, enthaltend $\omega$-3-Fettsäuren, insbesondere Eikosapentaensäure (EPA) oder ihre physiologisch unbedenklichen Ester als Bestandteile der Fettphase, die dadurch gekennzeichnet ist, daß die Fettemulsion

- diese $\omega$-3-Fettsäuren oder Ester in Reinform oder als Bestandteil von Fischölen,
- mittelkettige Triglyzeride (MCT),
- mindestens einen physiologisch unbedenklichen Emulgator,
- gegebenenfalls mindestens ein $\omega$-6-Fettsäuren lieferndes Pflanzenöl,
- $\alpha$-Tocopherol oder physiologisch unbedenkliche $\alpha$-Tocopherolester, sowie
- übliche Zusatz- und Hilfsstoffe

enthält, wobei

- das Verhältnis von EPA oder ihren physiologisch unbedenklichen Estern zu MCT zwischen 1 : 9 und 3 : 5 liegt,
- der gesamte Fettgehalt zwischen 5 und 30 % liegt,
- der Emulgatorgehalt zwischen 5 und 12 % (bezogen auf den Fettgehalt) liegt und
- der Gehalt an $\omega$-6-Fettsäuren liefernden Pflanzenölen zwischen 0 und 30 % (bezogen auf den Fettgehalt) liegt.

Der Einfluß von Eikosanoiden, die aus $\omega$-3-Fettsäuren, vor allem der Eikosapentaensäure, gebildet werden, auf Eikosanoide aus der Arachidonsäure kann sowohl unterstützend als auch hemmend sein: unterstützt wird z.B. die vasodilatorische und antiaggregatorische Wirkung des Prostazyklins ($PGI_2$) aus der $\omega$-6-Reihe durch das analoge $PGI_3$ aus der $\omega$-3-Reihe, das ein ähnliches Wirkprofil besitzt. Dagegen wird der vasokonstriktorische und proaggregatorische Effekt von $TXA_2$ ($\omega$-6) durch $TXA_3$ ($\omega$-3), das biologisch nicht aktiv ist, kompetitiv gehemmt, und $PGE_3$ ($\omega$-3), selbst weitgehend inaktiv, inhibiert die bronchokonstriktorischen, ödemfördernden und immunsuppressiven Eigenschaften von $PGE_2$ ($\omega$-6).

Ähnliche Interaktionen wie bei den Prostaglandinen und Thromboxanen treten auch zwischen den Leukotrienen aus der $\omega$-6- bzw. $\omega$-3-Fettsäurenreihe auf. $LTB_4$ ($\omega$-6) beipsielsweise ist ein hochpotenter Entzündungsmediator, während $LTB_5$ ($\omega$-3) nur geringe inflammatorische Aktivität zeigt und die Wirkung von $LTB_4$ infolgedessen abschwächt.

Sowohl $\omega$-6- als auch $\omega$-3-Fettsäuren können vom menschlichen Organismus nicht selbst gebildet sondern müssen von außen zugeführt werden, um ihren Bestand im Körper zu sichern. Bei den in Europa und Amerika üblichen Kostformen, der sogenannten "western diet", überwiegen $\omega$-6-Fettsäuren bei weitem; auch in der künstlichen enteralen wie parenteralen Ernährung dominieren $\omega$-6-Fettsäuren, die infolgedessen auch den Hauptanteil der Fettsäuren in den Membranphospholipiden des Körpers ausmachen. Bei einer Stimulation des Eikosanoidstoffwechsels durch die eingangs erwähnten Noxen überwiegen daher auch diejenigen Eikosanoidwirkungen, die die Entwicklung von Lungenerkrankungen begünstigen.

Die Kenntnis von den in pathologischen Situationen nachteiligen Wirkungen der $\omega$-6-Fettsäuren und von ihrer möglichen Antagonisierung durch $\omega$-3-Fettsäuren hat zu diätetischen Empfehlungen für die unterstützende Behandlung solcher Erkrankungen geführt, in deren Pathogenese Eikosanoide Mediatorfunktion besitzen. Auch für die parenterale Ernährung, in deren Rahmen bisher nur Fettemulsionen mit relativ hohem Anteil von $\omega$-6-Fettsäuren und sehr geringem Gehalt von $\omega$-3-Fettsäuren zum Einsatz gelangten, wurde eine Änderung dieses Verhältnisses zugunsten des $\omega$-3-Fettsäurenanteils empfohlen (W. Alexander, Arch. Surg. 121 (1986) 966-972). Dies vor allem deswegen, weil gerade bei Patienten mit absoluter Indikation für parenterale Ernährung, also polytraumatisierten Patienten, Patienten mit Sepsis, Patienten im Streßstoffwechsel, stets das Risiko eines Lungenversagens droht, ein Risiko, das durch den hohen Gehalt von $\omega$-6-Fettsäuren in den handelsüblichen Fettemulsionen und der damit verbundenen Stimulierung des Arachidonsäuremetabolismus noch verstärkt wird.

Tierexperimentelle Untersuchungen haben gezeigt, daß es durch Verabreichung von $\omega$-3-Fettsäuren im Gegensatz zu $\omega$-6-Fettsäuren tatsächlich möglich ist, die im Endotoxinschock gefährdete Lungenfunktion zu verbessern, die Sauerstoffversorgung und die Homöostase der Blutgase aufrechtzuerhalten und die Mortalität zu senken. Demgegenüber zeigten die Tiere, die eine $\omega$-6-fettsäurenreiche Diät erhielten, eine metabolische Azidose und höhere Mortalität bzw. kürzere Überlebenszeit (E. Mascioli et al., Clin. Nutr. 4, Special Supplement (1985) 99; E. Mascioli et al., JPEN 11/1, Supplement (1987) 23S; J.J. Pomposelli et al., JPEN 12/1, Supplement (1988) 9S). Bemerkenswert ist, daß solche Befunde sowohl unter enteraler als auch unter parenteraler Zufuhr von $\omega$-3-Fettsäuren bzw. $\omega$-6-Fettsäuren als Kontrolle erhalten wurden.

In den betreffenden Untersuchungen war es allerdings erforderlich, die $\omega$-3-Fettsäuren über einen längeren Zeitraum zu verabreichen, um die genannten Wirkungen zu erzielen. Dieser Umstand schränkt den therapeutischen Wert der enteralen oder parenteralen Verabreichung von ω-3-Fettsäuren zur Behandlung von Lungenkrankheiten ein. Es besteht statt dessen ein Bedarf an einer Darreichungsform, die die therapeutische Wirkung von ω-3-Fettsäuren schneller zur Geltung kommen läßt als bei den bisherigen Anwendungen.

Der vorliegenden Erfindung liegt somit die Aufgabe zugrunde, die Verwendung einer neuartigen Fettemulsion zur Herstellung eines Arzneimittels zur endotrachealen Behandlung von Lungenerkrankungen bereitzustellen.

Im Gegensatz zu allen bisherigen therapeutischen Ansätzen zur Behandlung von Lungenerkrankungen, die auf der systemischen Zufuhr von Wirkstoffen basieren, und im Gegensatz zu den bisherigen Vorschlägen zur Verabreichung von ω-3-Fettsäuren, wurde überraschenderweise gefunden, daß eine hervorragende protektive Wirkung auf die Lunge vor den Folgen eines Endotoxinschocks stets dann erzielt wird, wenn im Tierversuch beispielsweise Ratten mit der Fettemulsion zur endotrachealen Applikation behandelt werden.

Die vorliegende Erfindung betrifft somit die Verwendung einer Fettemulsion zur Herstellung eines Arzneimittels zur endotrachealen Behandlung von Lungenerkrankungen, enthaltend ω-3-Fettsäuren, insbesondere Eikosapentaensäure (EPA), bzw. ihre physiologisch unbedenklichen Ester als Bestandteile der Fettphase, wobei die Fettemulsion

- ω-3-Fettsäuren, insbesondere EPA, bzw. ihre physiologisch unbedenklichen Ester in Reinform oder als Bestandteil von Fischölen und/oder Fischölfraktionen,
- mindestens einen physiologisch unbedenklichen Emulgator,
- gegebenenfalls weitere Fette wie mittelkettige Triglyzeride (MCT),
- gegebenenfalls α-Tocopherol oder physiologisch unbedenkliche α-Tocopherolester
- gegebenenfalls Ascorbinsäure oder physiologisch unbedenkliche Ascorbinsäureester sowie
- übliche Zusatz- und Hilfsstoffe

enthält, wobei

- der gesamte Fettgehalt zwischen 2 und 20 % liegt und
- der Emulgatorgehalt zwischen 5 und 20 % (bezogen auf den Fettgehalt) liegt.

ω-3-Fettsäuren, insbesondere aber Eikosapentaensäure (EPA), bzw. ihre physiologisch unbedenklichen Ester können erfindungsgemäß entweder in Reinform oder als Bestandteil von Fischölen und/oder Fischölfraktionen verwendet werden.

Physiologisch und pharmakologisch unbedenklich sind niedere Alkylester bzw. Glyzerinester der ω-3-Fettsäuren, insbesondere der EPA. Bevorzugt sind die Ethylester und Triacylglyzerole.

Geeignete Fischöle sind beispielsweise solche, wie sie technisch in bedeutendem Umfang aus Kaltwasserfischen gewonnen werden. Bevorzugt sind hochgereinigte Fischölkonzentrate oder Fischölfraktionen, die beispielsweise aus Makrele, Sardine, Hering oder Lachs gewonnen werden, wobei diese einen EPA-Gehalt von vorzugsweise mindestens 25 % (bezogen auf die Fettsäuremethylester des Fischölkonzentrats) besitzen.

Als Eventualkomponente "weitere Fette" werden erfindungsgemäß mittelkettige Triglyzeride verwendet, die zu mindestens 90 % aus Glyzeriden der Caprylsäure und Caprinsäure bestehen. Der Gehalt dieser Komponenten liegt (bezogen auf den lipophilen Anteil der Emulsion) bei 0 bis 90 %.

Als Emulgatoren werden physiologisch unbedenkliche Emulgatoren wie Phospholipide tierischen und pflanzlichen Ursprungs verwendet, insbesondere solche aus Hühnereigelb oder Soja.

Der Emulgatorgehalt beträgt 5 bis 20 % (bezogen auf den Fettgehalt) und der gesamte Fettgehalt der Fettemulsion beträgt zwischen 2 und 20 %.

Als Emulgierhilfsstoffe können weiterhin Natriumsalze langkettiger Fettsäuren (bevorzugt in einer Konzentration von 0,005 bis 0,1 Gew.-%, bezogen auf die Gesamtemulsion) und/oder Cholesterin oder Cholesterinester (bevorzugt 0,005 bis 0,1 Gew.-%, bezogen auf die Gesamtemulsion) verwendet werden.

Die Fettemulsion weist vorzugsweise einen pH-Wert im Bereich von 6 bis 9 auf.

Weiterhin kann der Fettemulsion gegebenenfalls α-Tocopherol oder α-Tocopherol-Ester in einer Menge von 0 bis 100 mg, bezogen auf 100 g Fett, zugegeben werden, insbesondere wenn ω-3-Fettsäuren, insbesondere EPA, bzw. ihre Ester in Reinform verwendet werden.

Schließlich kann der Fettemulsion gegebenenfalls außerdem Ascorbinsäure oder physiologisch unbedenkliche Ascorbinsäureester in einer Menge von 0 bis 500 mg, bezogen auf 100 g Fett, zugegeben werden.

Die Fettemulsion wird endotracheal, vorzugsweise bei respiratorischer Insuffizienz, inhalativen Noxen, chronisch obstruktiven Lungenerkrankungen und Aspirationspneumonie verwendet. Weiterhin verwendet man die Fettemulsion vorzugsweise zur endotrachealen Verabreichung nutritiv und pharmakologisch wirksamer ω-3-Fettsäuren bei vorstehenden und nachstehenden Indikationen und setzt die Fettemulsion insbesondere bei den vorstehend genannten Indikationen zur Verbesserung der Splanchnikusperfusion und damit zur Steigerung der Klärfunktion des RES ein.

In dem betreffenden, unten näher beschriebenen Experiment wurde durch die Infusion von Endotoxin bei Ratten eine Schocksituation erzeugt. An den beiden der Endotoxininfusion vorangehenden Tagen waren die Tiere auf endotrachealem Weg mit entweder einer ω-3-fettsäurenhaltigen Emulsion (EPA-Ethylester) oder einer ω-6-fettsäurenhaltigen Emulsion (Intralipid[R]) bzw. mit Kochsalzlösung (als Plazebo) behandelt worden. Der Endotoxinschock hatte in der Gruppe mit ω-3-Fettsäuren eine signifikant geringere Mortalität als in der Kochsalz-Gruppe und in der Gruppe mit ω-6-Fettsäuren zur Folge. Im Gegensatz zu der Plazebo-Gruppe und der Vergleichsgruppe mit ω-6-Fettsäuren zeigten die Tiere mit ω-3-Fettsäuren nur geringe Symptome einer respiratorischen Insuffizienz, und der histologische Befund der Lunge blieb weitgehend normal. Bei der Gruppe mit ω-3-Fettsäuren war die Konzentration von $TXB_2$, einem stabilen Metaboliten des aus Arachidonsäure gebildeten Thromboxan $A_2$, in der Lunge signifikant geringer als bei den beiden anderen Gruppen, ebenso wie die Konzentrationen von $PGE_2$ und $PGF_{2a}$, die ebenfalls beide aus Arachidonsäure entstehen. Dies beweist die erfolgreiche Hemmung der Arachidonsäuremetabolite durch die erfindungsgemäße Verabreichung von ω-3-Fettsäuren und erklärt die bessere Resistenz der mit Eikosapentaensäureethylester behandelten Tiere gegenüber den Folgen des Endotoxinschocks. Diese Resistenz ergibt sich aus der eikosapentaensäureinduzierten Modulation des Wirkungsspektrums von Arachidonsäuremetaboliten, die - wenn sie nicht wie bei der beschriebenen Erfindung antagonisiert werden - die Ausbildung eines Lungenversagens neben anderen Schockreaktionen fördern.

Gegenüber allen anderen Anwendungsarten zeichnet sich die erfindungsgemäße endotracheale Anwendung von ω-3-Fettsäuren bei Erkrankungen der Lunge dadurch aus, daß die Arachidonsäureantagonisten akut und unmittelbar an das Zielorgan herangebracht werden. Auf diese Weise lassen sich mit geringeren Dosen als bei systemischer (intravenöser oder oraler) Anwendung höhere Wirkspiegel am Ort des Krankheitsgeschehens erreichen, systemische Nebenwirkungen, z.B. eine Hemmung der Prostazyklin-Produktion, lassen sich vermeiden, die Dosierung kann besser an der Wirkung orientiert und variabler gesteuert werden. Außerdem lassen sich eventuell bestehende Störungen der Diffusion vom Gefäß zum Lungengewebe umgehen. Bei bereits beatmungspflichtigen Patienten besteht die Möglichkeit, ω-3-Fettsäurenester direkt oder mittels Vernebler in den Tubus zu applizieren. Bei nicht beatmeten Patienten läßt sich der Wirkstoff als Aerosol (Inhalationsspray) verabreichen. Derartige Dosieraerosole werden nach allgemein bekannter Technik mit den in dem Abschnitt "Herstellungsbeispiele" näher beschriebenen Emulsionen und den üblicherweise verwendeten Treibgasen/Treibgasgemischen befüllt.

Die Erfindung wird im folgenden durch Anwendungsbeispiele und Herstellungsbeispiele näher erläutert. Bei den Prozentangaben in der Beschreibung und in den Patentansprüchen handelt es sich, sofern nichts Anderes angegeben, um solche in Gramm pro 100 ml Lösung.

Die Wirkung der erfindungsgemäßen Verwendung der Fettemulsion wird durch folgende Untersuchung näher erläutert:

30 Ratten wurden einzeln in Stoffwechselkäfigen bei einer Raumtemperatur von 22 °C und einer Luftfeuchtigkeit von 70 % gehalten sowie mit Wasser und Standardfutter ad libitum versorgt. Nach dreitägiger Eingewöhnungsphase wurden die Tiere randomisiert drei Behandlungsgruppen zugeteilt:

Gruppe I: (Plazebogruppe) Behandlung mit physiologischer Kochsalzlösung (Tagesdosis: 10 ml/kg KG)

Gruppe II: Behandlung mit einer 10 %igen erfindungsgemäßen Emulsion von Eikosapentaensäureethylester gemäß Herstellungsbeispiel 1 (Tagesdosis: 10 ml/kg KG)

Gruppe III: Behandlung mit 10 % Intralipid[R]
Zusammensetzung: 100 g fraktioniertes Sojaöl, 12 g fraktioniertes Eilecithin und 22,5 g Glycerin, mit destilliertem Wasser auf 1 000 ml ergänzt
(Tagesdosis: 10 ml/kg KG).

Die Testsubstanzen wurden den Ratten in vier Einzeldosen/Tag an zwei aufeinanderfolgenden Tagen per injectionem über eine Sonde in die Trachea verabreicht. 14 h nach der letzten Injektion erhielten sämtliche Tiere über die Schwanzvene eine achtstündige Endotoxin-Infusion ($0,7$ mg $\cdot$ kg$^{-1}$ $\cdot$ h$^{-1}$; Kontroll-Standard-Endotoxin von Difco, Detroit). Unmittelbar nach der Endotoxin-Infusion wurden die Tiere getötet und seziert.

Während der Endotoxininfusion fielen die Tiere der Gruppe I und insbesondere der Gruppe III durch drastisch erhöhte Atemfrequenz und Atemtiefe auf; der Kreislauf war zentralisiert; aus der Nase dieser Tiere trat schaumiges Exsudat.

Demgegenüber ließen die Ratten, die mit der erfindungsgemäß verwendeten Fettemulsion auf Basis von Eikosapentaensäureethylester behandelt worden waren (Gruppe II), nur eine geringe Ventilationssteigerung bei weitgehendem Fehlen der übrigen Zeichen erkennen.

Im Vergleich zu der Plazebogruppe (Gruppe I) lag das Lungengewicht bei der Sektion in Gruppe II (Eikosapentaensäureethylester) um durchschnittlich 15 % niedriger und in Gruppe III um durchschnittlich 24 % höher.

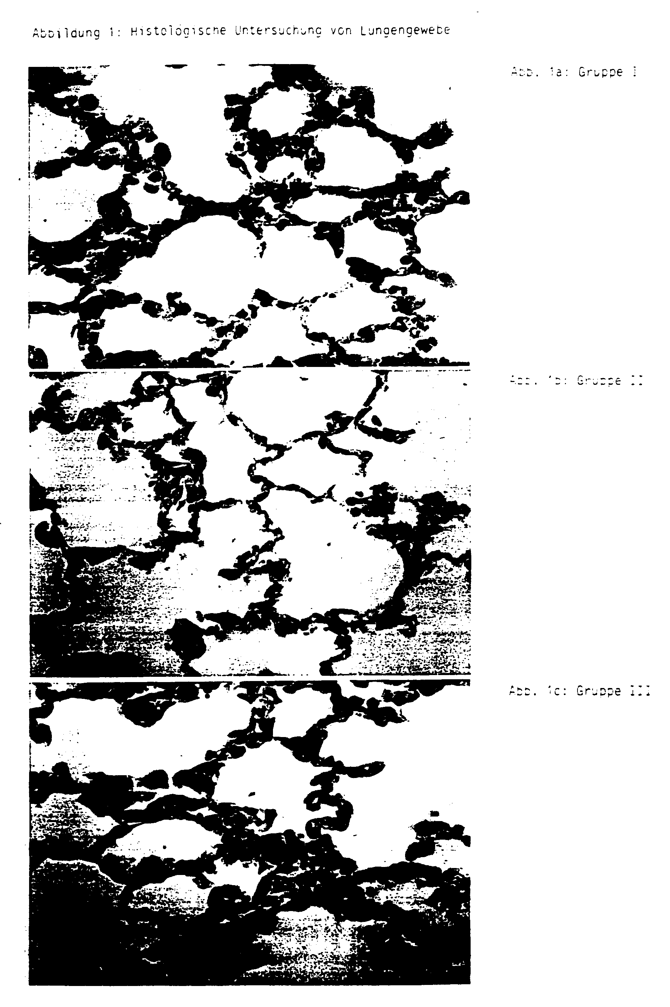

Die histologische Untersuchung von hämatoxylin- und eosingefärbten Lungenschnitten ergab in der Gruppe II einen weitgehend normalen Befund; in den Gruppen I und III wurden dagegen ödematös geschwollene Alveolen mit ausgeprägter leukozytärer Infiltration der Zellen vorgefunden (s. Anlage, Abb. 1a-c).

Nach Homogenisation und Zentrifugation von Lungengewebe, das bei der Sektion entnommen worden war, wurden im Zentrifugationsüberstand mittels handelsüblicher Radioimmunoassay-Kits die Konzentrationen ausgewählter Mediatoren aus dem Prostaglandinstoffwechsel ($TXB_2$, $PGF_{2a}$, $PGE_2$) bestimmt.

Konzentration der Prostaglandin-Mediatoren im Lungenhomogenat (MW ± SD; pg/ml):

|  | $TXB_2$ | $PGF_{2a}$ | $PGE_2$ |
|---|---|---|---|
| Gruppe I: | 22,1 ± 3,8 | 11,0 ± 3,3 | 7,1 ± 1,7 |
| Gruppe II: | 9,9 ± 3,1[1] | 5,9 ± 2,0[1] | 3,4 ± 1,6[1] |
| Gruppe III: | 30,9 ± 5,8 | 15,3 ± 3,3 | 9,2 ± 2,8 |

[1] $p < 0,01$ vs. Gr. I und Gr. III

$TXB_2$, ein stabiler Metabolit des stark vasokontriktorischen $TXA_2$, zeigte in der Gruppe II die signifikant niedrigsten Konzentrationen, ebenso das zur pulmonalen Gefäßverengung führende $PGF_{2a}$ sowie das bronchokonstriktorische und immunsuppressive $PGE_2$. Alle drei Eikosanoide entstammen der Arachidonsäure ($\omega$-6) und spielen eine entscheidende Rolle bei der Pathogenese des Lungenversagens (siehe Einleitung). Die geringeren Konzentrationen dieser drei Mediatoren in Gruppe II beweisen die erfolgreiche Hemmung des Arachidonsäurestoffwechsels durch die zugeführten $\omega$-3-Fettsäuren und die Wirksamkeit der erfindungsgemäßen Verabreichung von $\omega$-3-fettsäurenhaltigen Emulsionen.

Kurz vor der Tötung der Tiere wurden außerdem Blutproben zur Bestimmung der Glukosekonzentration entnommen:

Blutglukose (MW ± SD; mg/ml)

| Gruppe I | Gruppe II | Gruppe III |
|---|---|---|
| 108 ± 12[1] | 101 ± 10[2] | 126 ± 16 |

[1] $p < 0,005$ vs. Gruppe III
[2] $p < 0,001$ vs. Gruppe III

Die Glukosekonzentration lag unter den endotracheal verabreichten erfindungsgemäßen $\omega$-3-Fettsäuren am niedrigsten. Der Anstieg des Blutzuckers in den beiden anderen Gruppen ist Ausdruck einer verstärkten hepatischen Glukoseproduktion, die ihrerseits die Folge einer unter dem vasokonstriktorischen Einfluß von $TXA_2$ und $PGF_{2a}$ verminderten Splanchnikusperfusion darstellt. Unter dem vasodilatorischen Einfluß von Eikosapentaensäure nimmt dagegen die Splanchnikusperfusion zu und die Glukosehomöostase normalisiert sich. Aus pathophysiologischer Sicht ist die Verbesserung der Splanchnikusperfusion auch deswegen von Bedeutung, weil dadurch die normale Klärfähigkeit der Leber für Bakterien und Toxine wiederhergestellt wird und deren negative Sekundärwirkungen auf die Lunge verhindert werden.

Herstellungsbeispiele

Beispiel 1:

Eine Mischung aus 100 g Eikosapentaensäureethylester, 12,0 g gereinigten Eiphospholipiden, 100 mg $\alpha$-Tocopherol und 0,2 g Natriumstearat wird mittels eines Ultra-Turrax fein dispergiert. Mit Aqua ad iniectabilia, das 25,0 g Glycerol und 0,5 mmol NaOH enthält, wird unter Rühren auf 1,0 l aufgefüllt. Diese grobe Voremulsion wird in einem Hochdruckhomogenisator bei einem Druck von 400 kg/cm² homogenisiert. Nach Abfüllung in Glasvials geeigneter Qualität wird nach allgemein bekannten Verfahren hitzesterilisiert. Die Teilchen der sterilen Emulsion sind kleiner als 1 μm.

Beispiel 2:

Eine Mischung aus 40 g Eikosapentaensäureethylester, 60 g MCT, 12 g gereinigten Eiphospholipiden, 50 mg α-Tocopherol und 0,2 g Natriumstearat wird, wie unter Beispiel 1 beschrieben, dispergiert und nach Auffüllen mit Aqua ad iniectabilia (enthält 50 g Glycerol) auf 2 l homogenisiert, abgefüllt und sterilisiert. Die Emulsionstropfen sind kleiner als 1 μm.

Beispiel 3:

Eine Mischung aus 300 g hochgereinigtem Fischöl, 15 g gereinigten Sojaphospholipiden, 100 mg α-Tocopherol, 300 mg Ascorbylpalmitat und 3 g Natriumstearat wird, wie unter Beispiel 1 beschrieben, dispergiert und nach Auffüllen auf 2 l mit Aqua ad iniectabilia (enthält 50 g Glycerol) homogenisiert, abgefüllt und sterilisiert. Die Emulsionströpfchen sind kleiner als 1 μm.

**Patentansprüche**

1. Verwendung einer Fettemulsion, enthaltend ω-3-Fettsäuren, insbesondere Eikosapentaensäure (EPA), bzw. ihre physiologisch unbedenklichen Ester als Bestandteile der Fettphase, wobei die Fettemulsion
   - ω-3-Fettsäuren, insbesondere EPA, bzw. ihre physiologisch unbedenklichen Ester in Reinform oder als Bestandteil von Fischölen und/oder Fischölfraktionen,
   - mindestens einen physiologisch unbedenklichen Emulgator,
   - gegebenenfalls weitere Fette wie mittelkettige Triglyzeride (MCT),
   - gegebenenfalls α-Tocopherol oder physiologisch unbedenkliche α-Tocopherolester,
   - gegebenenfalls Ascorbinsäure oder physiologisch unbedenkliche Ascorbinsäureester sowie
   - übliche Zusatz- und Hilfsstoffe enthält, wobei
   - der gesamte Fettgehalt zwischen 2 und 20 % liegt und
   - der Emulgatorgehalt zwischen 5 und 20 % (bezogen auf den Fettgehalt) liegt,
   zur Herstellung eines Arzneimittels zur endotrachealen Behandlung von Lungenerkrankungen.

2. Verwendung gemäß Anspruch 1 zur prophylaktischen und therapeutischen Behandlung von respiratorischer Insuffizienz.

3. Verwendung gemäß Anspruch 1 zur Behandlung von inhalativen Noxen wie beispielsweise Rauchvergiftungen und Verbrennungen der Atemwege.

4. Verwendung gemäß Anspruch 1 zur Behandlung bei chronisch obstruktiven Lungenerkrankungen wie beispielsweise Asthma und chronischer Bronchitis.

5. Verwendung gemäß Anspruch 1 zur prophylaktischen und therapeutischen Behandlung der Aspirationspneumonie.

6. Verwendung gemäß Anspruch 1 zur Verbesserung der Splanchnikusperfusion und damit der Steigerung der Klärfunktion des RES.

7. Verwendung gemäß Anspruch 1 zur endotrachealen Verabreichung nutritiv und pharmakologisch wirksamer ω-3-Fettsäuren bei den Indikationen nach Ansprüchen 1 bis 6.

8. Verwendung gemäß Ansprüchen 1 bis 7, dadurch gekennzeichnet, daß das Fischöl ein hochgereinigtes Fischölkonzentrat mit einem Gehalt an EPA von mindestens 25 % (bezogen auf die Fettsäuremethylester des Fischölkonzentrats) ist.

9. Verwendung gemäß Ansprüchen 1 bis 8, dadurch gekennzeichnet, daß dieses Fischöl/Fischölkonzentrat erhältlich ist durch Verarbeitung von Kaltwasserfischen, beispielsweise von Makrele, Sardine, Hering oder Lachs.

**10.** Verwendung gemäß Ansprüchen 1 bis 9, dadurch gekennzeichnet, daß die als Eventual-Fettkomponenten eingesetzten mittelkettigen Triglyceride zu mindestens 90 % aus Glyzeriden der Caprylsäure ($C_8$) und Caprinsäure ($C_{10}$) bestehen und der Gehalt dieser Komponente 0 bis 90 % (bezogen auf den lipophilen Anteil) beträgt.

**11.** Verwendung gemäß Ansprüchen 1 bis 10, dadurch gekennzeichnet, daß der eingesetzte Emulgator ein Phospholipid tierischen oder pflanzlichen Ursprungs, insbesondere aus Hühnereigelb oder Sojabohne, ist.

**12.** Verwendung gemäß Ansprüchen 1 bis 11, dadurch gekennzeichnet, daß sie weitere Emulgierhilfsstoffe wie Natriumsalze langkettiger Fettsäuren ( bevorzugt 0, 005 bis 0, 1 Gew. -%, bezogen auf die Gesamtemulsion ) und/oder Cholesterin oder Cholesterinester (bevorzugt 0,005 bis 0,1 Gew.-%, bezogen auf die Gesamtemulsion ) enthält.

**13.** Verwendung gemäß Ansprüchen 1 bis 12, dadurch gekennzeichnet, daß sie 0 bis 100 mg $\alpha$-Tocopherol oder $\alpha$-Tocopherolester, bezogen auf 100 g Fett, enthält.

**14.** Verwendung gemäß Ansprüchen 1 bis 13, dadurch gekennzeichnet, daß sie 0 bis 500 mg Ascorbinsäure oder Ascorbinsäureester, bezogen auf 100 g Fett, enthält.

**15.** Verwendung gemäß Ansprüchen 1 bis 14, dadurch gekennzeichnet, daß sie einen pH-Wert im Bereich von 6 bis 9 aufweist.

## Claims

**1.** Use of a fat emulsion containing $\omega$-3 fatty acids, and more specifically eicosapentaenoic acid (EPA) or its physiologically acceptable esters as components of the fat phase,
wherein the fat emulsion contains
- $\omega$-3 fatty acids, and especially EPA, and/or its physiologically acceptable esters in the pure state or as component(s) of fish oils and/or fish oil fractions,
- at least one physiologically acceptable emulsifier,
- optionally further fats such as medium-chain triglycerides (MCT),
- optionally $\alpha$-tocopherol or physiologically acceptable $\alpha$-tocopherol esters,
- optionally ascorbic acid or physiologically acceptable ascorbic acid esters, as well as
- conventional additives or auxiliary materials,
whereby
- the total fat content is between 2 and 20%, and
- the emulsifier content is between 5 and 20% (based on the fat content),
for preparing a medicament for the endotracheal treatment of pulmonary diseases.

**2.** The use according to claim 1 for the prophylactic and therapeutic treatment of respiratory insufficiency.

**3.** The use according to claim 1 for the treatment of inhalative noxae such as, for example, smoke poisoning and burns of the respiratory tract.

**4.** The use according to claim 1 for chronic obstructive pulmonal diseases such as, for example, asthma and chronic bronchitis.

**5.** The use according to claims 1 for the prophylactic and therapeutical treatment of aspiration pneumonia.

**6.** The use according to claims 1 for improving the splanchnic perfusion and, hence, the clearing function of the RES.

**7.** The use according to claim 1 for the endotracheal application of nutritively and pharmacologically effective $\omega$-3 fatty acids upon the indications according to claims 1 to 6.

**8.** The use according to claims 1 to 7, characterized in that said fish oil is a highly purified fish oil concentrate having an EPA content of at least 25% (based on the fatty acid methyl esters of the fish oil concentrate).

9.  The use according to claims 1 to 8, characterized in that said fish oil/fish oil concentrate is obtainable by processing cold water fish such as, for example, mackerel, sardine, herring or salmon.

10. The use according to claims 1 to 9, characterized in that of the medium-chain triglycerides employed as optional components at least 90% consist of glycerides of caprylic ($C_8$) and capric ($C_{10}$) acids and the amount of this component is from 0 to 90% (relative to the lipophilic proportion).

11. The use according to claims 1 to 10, characterized in that the emulsifier employed is a phospholipid of animal or vegetable origin, and more particularly one derived from hen's egg yolk or soybean.

12. The use according to claims 1 to 11, characterized in that it contains further emulsifying auxiliary materials such as sodium salts of long-chain fatty acids (preferably in a concentration of from 0.005 to 0.1% by weight, based on the total emulsion) and/or cholesterol or cholesterol esters (preferably in a concentration of from 0.005 to 0.1% by weight, based on the total emulsion).

13. The use according to claims 1 to 12, characterized in that it contains $\alpha$-tocopherol or $\alpha$-tocopherol ester in an amount of from 0 to 100 mg, based on 100 g of fat.

14. The use according to claims 1 to 13, characterized in that it contains ascorbic acid or ascorbic acid ester in an amount of from 0 to 500 mg, based on 100 g of fat.

15. The use according to claims 1 to 14, characterized in that it has a pH value within the range of from 6 to 9.

**Revendications**

1.  Utilisation d'une émulsion de matière grasse contenant des acides gras $\omega$-3, notamment l'acide éicosapentaénoïque (EPA), ou leurs esters physiologiquement neutres, comme constituants de la phase grasse, cette émulsion (de matière) grasse contenant
    - des acides gras $\omega$-3, notamment EPA, ou leurs esters physiologiquement neutres, sous forme pure ou sous forme de constituants d'huile de poissons et/ou de fractions d'huile(s) de poisson,
    - au moins un émulsifiant physiologiquement neutre,
    - éventuellement d'autres matières grasses comme des triglycérides à chaînes moyennes ("MCT"),
    - éventuellement de l'$\alpha$-tocophérol ou des esters physiologiquement neutres d'$\alpha$-tocophérol,
    - éventuellement de l'acide ascorbique ou des esters physiologiquement neutres de l'acide ascorbique, ainsi que
    - des additifs et adjuvants usuels,
        la teneur totale en matière grasse se situant entre 2 et 20%, et
        la teneur en émulsifiant se situant entre 5 et 20% par rapport à la teneur en matière grasse, pour la fabrication d'un médicament destiné à traiter par voie endotrachéale les maladies des poumons.

2.  Utilisation selon la revendication 1 pour le traitement prophylactique et thérapeutique d'une insuffisance respiratoire.

3.  Utilisation selon la revendication 1 pour le traitement d'agents pathogènes inhalés, comme par exemple des empoisonnements par des fumées et des brûlures des voies respiratoires.

4.  Utilisation selon la revendication 1 pour le traitement de maladies d'obstruction chronique des poumons, comme par exemple l'asthme et de la bronchite chronique.

5.  Utilisation selon la revendication 1 pour le traitement prophylactique et thérapeutique de la pneumonie par aspiration.

6.  Utilisation selon la revendication 1 pour améliorer la perfusion splanchnique et donc augmenter la fonction de clarification (ou "clairance") du RES (système réticuloendothélial).

7.  Utilisation selon la revendication 1 pour une administration endotrachéale d'acides gras $\omega$-3, à action nu-

tritive et pharmacologique, selon les indications des revendications 1 à 6.

8. Utilisation selon les revendications 1 à 7, caractérisée en ce que l'huile de poisson est un concentré hautement purifié d'huile de poisson, ayant une teneur en EPA d'au moins 25% (par rapport à l'ester méthylique d'acide gras du concentré d'huile de poisson).

9. Utilisation selon les revendications 1 à 8, caractérisée en ce que cette huile de poisson/ce concentré d'huile de poisson peut être obtenu(e) par traitement de poissons d'eau froide, par exemple des maquereaux, des sardines, du hareng ou du saumon.

10. Utilisation selon les revendications 1 à 9, caractérisée en ce que les triglycérides à chaînes moyennes éventuellement utilisés comme constituants gras, consistent pour au moins 90% en des glycérides de l'acide caprylique ($C_8$) et de l'acide caprique (en $C_{10}$) et en ce que la teneur en ces constituants représente 0 à 90°% (par rapport à la fraction lipophile).

11. Utilisation selon les revendications 1 à 10, caractérisée en ce que l'émulsifiant utilisé est un phospholipide d'origine animale ou végétale, provenant en particulier de jaune d'oeuf de poule ou de haricots de soja.

12. Utilisation selon les revendications 1 à 11, caractérisée en ce que l'émulsion contient d'autres adjuvants d'émulsification, comme des sels de sodium d'acides gras à longues chaînes (de préférence 0,005 à 0,1 en poids, par rapport à l'émulsion totale) et/ou du cholestérol ou un ester de cholestérol (de préférence 0,005 à 0,1% en poids, par rapport à l'émulsion totale).

13. Utilisation selon les revendications 1 à 12, caractérisée en ce que l'émulsion contient 0 à 100 mg d'$\alpha$-tocophérol ou d'un ester d'$\alpha$-tocophérol, pour 100g de matière grasse.

14. Utilisation selon les revendications 1 à 13, caractérisée en ce que l'émulsion contient 0 à 500 mg d'acide ascorbique ou d'un ester de l'acide ascorbique, pour 100 g de matière grasse.

15. Utilisation selon les revendications 1 à 14, caractérisée en ce que l'émulsion présente une valeur de pH se situant entre 6 et 9.

Abbildung 1: Histologische Untersuchung von Lungengewebe

Abb. 1a: Gruppe I

Abb. 1b: Gruppe II

Abb. 1c: Gruppe III